(19) Europäisches Patentamt
European Patent Office
Office européen des brevets

(11) EP 1 747 801 B1

(12) FASCICULE DE BREVET EUROPEEN

(45) Date de publication et mention de la délivrance du brevet:
**14.11.2012 Bulletin 2012/46**

(21) Numéro de dépôt: **06116467.9**

(22) Date de dépôt: **30.06.2006**

(51) Int Cl.:
***A61Q 3/02*** *(2006.01)* ***A61K 8/45*** *(2006.01)*
***A61K 8/86*** *(2006.01)* ***A61K 8/37*** *(2006.01)*
***A61K 8/87*** *(2006.01)* ***A61K 8/81*** *(2006.01)*

# (54) Vernis à ongles comprenant un polymère à chaîne(s) polyoxyalkylène

Nagellack enthaltend ein Polymer mit einer Polyoxyalkylenkette

Nail coating comprising a polymer with a polyalkylene chain

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **29.07.2005 FR 0508180**

(43) Date de publication de la demande:
**31.01.2007 Bulletin 2007/05**

(73) Titulaire: **L'Oréal**
**75008 Paris (FR)**

(72) Inventeurs:
- **Blin, Xavier**
  **75015 Paris (FR)**
- **Ilekti, Philippe**
  **94700 Maison-Alfort (FR)**

(74) Mandataire: **Duvert, Sandra**
**L'Oréal**
**D.I.P.I.**
**River PLaza**
**25-29 Quai Aulagnier**
**92600 Asnieres-sur-Seine (FR)**

(56) Documents cités:
**EP-A- 0 648 485** **EP-A- 0 658 609**
**EP-A- 1 495 748** **EP-A- 1 595 524**
**US-A- 4 104 333** **US-A- 4 229 227**
**US-A- 6 106 820** **US-B1- 6 737 069**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 747 801 B1

**Description**

La présente invention a pour objet un vernis à ongles comprenant un polymère comprenant au moins un bloc polyoxyalkylène. L'invention a également pour objet un procédé de maquillage ou de soin des ongles.

La composition de vernis à ongles peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles. Ces compositions peuvent s'appliquer sur les ongles d'êtres humains ou bien encore sur des faux ongles.

Les compositions de vernis à ongles comprennent en général des particules solides telles que les pigments, les nacres, les charges, qui sont en dispersion dans le milieu continu aqueux ou le milieu solvant organique de la composition.

Or ces particules ont tendance à sédimenter au cours du temps, du fait de leur densité qui est supérieure à celle du milieu continu dans lequel elles sont dispersées. Cette sédimentation se traduit par une modification de l'aspect macroscopique de la composition, et en particulier, dans le cas de vernis à ongles colorés, par une hétérogénéité de la couleur du vernis et une détérioration de la tenue du film de vernis sur les ongles.

Le brevet US 6,106,820 décrit par exemple des compositions cosmétiques, et notamment des vernis à ongles présentant une bonne tenue et une bonne résistance à l'écaillage comprenant des monomères simples oxyalkylènes (ie hexylène glycol et néopentyl glycol).

Le brevet US 4,104,333 décrit encore des compositions pour ongles artificiels comprenant des monomères (a) et (b) et un initiateur de polymérisation, lesdits monomères réticulant après application sur l'ongle pour former un film.

Le brevet US 4,229,227 concerne également des homopolymères polyéthylène glycol ou polypropylène glycol.

Afin d'améliorer la stabilisation des compositions, le formulateur dispose d'agents épaississants ou gélifiants tels que par exemple la bentone ou la silice ; toutefois, l'incorporation de ces agents en une quantité nécessaire à la stabilisation du vernis a tendance à matifier le film de vernis ce qui n'est pas souhaitable dans le cas des vernis à ongles où l'on cherche à obtenir un film brillant sur l'ongle.

La demande EP 1 495 748 utilise par exemple des dérivés de cellulose comme épaississants dans des compositions de vernis à ongles.

Le demandeur a découvert de façon surprenante que l'incorporation dans un vernis à ongles à milieu solvant organique, d'un polymère particulier comprenant au moins une chaîne polyoxyalkylène (appelé par la suite « polymère polyoxyalkyléné ») permet l'obtention d'une composition de vernis à ongles qui présente une bonne stabilité et une bonne homogénéité de la couleur dans le temps, ainsi que la formation d'un film sur les ongles brillant et présentant une bonne tenue dans le temps.

De façon plus précise, l'invention a pour objet une composition de vernis à ongles comprenant, dans un milieu cosmétiquement acceptable comprenant un milieu solvant organique, au moins 0,1% d'au moins un polymère selon la revendication 1 ayant un squelette soluble dans le milieu solvant organique, ledit polymère comprenant au moins une chaîne polyoxyalkylène dans sa chaîne principale et au moins un agent épaississant.

Par soluble, on entend que le polymère est soluble à une concentration d'au moins 1% en poids par rapport au poids total de la composition, dans le solvant majoritaire en poids du milieu solvant organique de la composition, de préférence dans un solvant ester court, à température ambiante (25°C) et pression atmosphérique ($10^5$ Pa).

Ces polymères polyoxyalkylénés particuliers permettent de stabiliser la composition selon l'invention et peuvent être formulés en complément d'un gélifiant classique comme la bentone ou la silice, sans avoir l'inconvénient de l'adjonction de grandes quantités de bentone qui entraîne une perte de brillance du film de vernis.

Par "milieu cosmétiquement acceptable", on entend au sens de l'invention un milieu non toxique et susceptible d'être appliqué sur la peau, les phanères ou les lèvres du visage d'êtres humains.

L'invention a aussi pour objet un procédé cosmétique de maquillage ou de soin non thérapeutique des ongles comprenant l'application sur les ongles d'au moins une couche d'une composition de vernis à ongles telle que définie ci-dessus.

L'invention a encore pour objet l'utilisation, dans une composition de vernis à ongles à milieu solvant organique, d'au moins au moins un polymère selon la revendication 1 soluble dans le milieu solvant organique, ledit polymère comprenant au moins une chaîne polyoxyalkylène dans sa chaîne principale ou sous forme de greffon, pour obtenir un vernis à ongles présentant une bonne stabilité et une bonne homogénéité de la couleur dans le temps, ainsi que la formation d'un film sur les ongles brillant et de bonne tenue.

## Polymère à chaîne(s) polyoxyalkylène

Le polymère à chaîne(s) polyoxyalkylène(s) de la composition selon l'invention est soluble dans le milieu solvant organique et comprend au moins une chaîne polyoxyalkylène dans sa chaîne principale selon la revendication 1.

Le polymère polyoxyalkyléné est choisi parmi les polymères ayant un squelette polyuréthane tel que défini ci-après.

On utilise des polymères de structure $R_9$-(ABA)n- $R_9$ dans laquelle :

les radicaux $R_9$ représentent indépendamment un atome d'hydrogène, un groupe alkyle ou aryle comprenant de 6 à 18 atomes de carbone,
A représente une chaîne polyoxylakylénée, de préférence comprenant des groupes polyoxyethylène (POE), une chaîne hydrocarbonée comprenant des groupes polyoxylakyléné, une chaîne de copoly(oxyethylène/oxypropylène),
B représente un groupement -CO-NH-$R_{10}$-NH-CO- dans lequel $R_{10}$ est une chaîne hydrocarbonée linéaire ou cyclique, de préférence un groupe alkyle comprenant de 4 à 12 atomes de carbone et
n est un entier allant de 1 à 10.

A titre de copolymère utilisable dans la composition selon l'invention, on peut notamment citer les polymères obtenus par réaction d'hexaméthylène diisocyanate et d'au moins un copolymère bloc polyoxyéthylène / polyoxypropylène comprenant des unités OE et OP comme par exemple le Poloxamer 184 (nom INCI) tel que celui commercialisé sous la référence SYNPERONIC PE L/64 par la société UNIQUEMA.

On peut aussi utiliser les polymères de formule suivante :

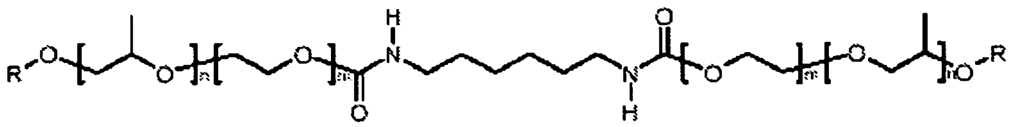

dans laquelle :

les radicaux R, identiques ou différents représentent un groupement alkyle comprenant de 6 à 20 atomes de carbone,
m représente un nombre entier allant de 1 à 100 et
n représente un nombre entier allant de 1 à 200.

Parmi les polymères répondant à cette formule, on peut citer en particulier les polymères de diuréthane (hexamethylène diisocyanate) d'alcools en C16-C18 oxyethylénés (66 OE) et oxypropylénés (14 OP) (Nom INCI : Polyethylène glycol-14 palmeth-60 hexyl dicarbamate) tel que celui commercialisé sous la référence ELFACOS T 212 par la socité Akzo Nobel.

Le polymère polyoxyalkyléné de la composition présente avantageusement un poids moléculaire supérieur ou égal à 1000 g/mol, allant par exemple de 1000 à 300 000 g/mol, de préférence supérieur ou égal à 1500 g/mol, allant par exemple de 1500 à 150 000 g/mol, mieux supérieur à 2000 g/mol, avantageusement de 2000 à 50 000 g/mol.

Le ou les copolymères polyoxyalkylénés sont présents en une teneur supérieure ou égale 0,1% en poids par rapport au poids total de la composition, de préférence supérieure ou égale à 0,5% en poids et mieux supérieure ou égale à 1% en poids.

Ces copolymères polyoxyalkylénés peuvent être présents en une quantité allant de 0,1 à 15% en poids par rapport au poids total de la composition, de préférence de 0,5 à 10%, mieux de 1 à 8% en poids et encore mieux de 1 à 5% en poids.

**Milieu solvant organique**

Le milieu cosmétiquement acceptable de la composition cosmétique selon l'invention comprend un milieu solvant organique comprenant un solvant organique ou un mélange de solvants organiques.

Le solvant organique peut être choisi parmi :

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la $\gamma$-butyrolactone ;
- les esters à chaîne courte (ayant de 3 à 8 atomes de carbone au total) tels que l'acétate d'éthyle, l'acétate de

méthyle, l'acétate de propyle, l'acétate d'isopropyl, l'acétate de n-butyle, l'acétate d'isopentyle, l'acétate de méthoxypropyle, le lactate de butyle ;

- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle ,
- les acétals tels que méthylal ;
- et leurs mélanges.

De préférence, le solvant est choisi parmi les esters à chaîne courte ayant de 3 à 8 atomes de carbone au total, tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle et leurs mélanges.
Le milieu solvant organique peut représenter de 10 à 95% en poids, par rapport au poids total de la composition, de préférence de 15% à 80% en poids, et mieux de 20 à 60% en poids.
La composition selon l'invention peut éventuellement comprendre un milieu aqueux, dans ce cas, le milieu aqueux est présent en une teneur inférieure ou égale à 2% en poids par rapport au poids total de la composition, de préférence inférieure ou égale à 1% en poids.

Polymère filmogène

La composition peut comprendre avantageusement un polymère filmogène.
Selon la présente invention, on entend par "polymère filmogène", un polymère apte à former à lui seul ou en présence d'un agent auxiliaire de filmification, un film continu sur un support, notamment sur les matières kératiniques.
Parmi les polymères filmogènes utilisables dans la composition de la présente invention, on peut citer les polymères synthétiques, de type radicalaire ou de type polycondensat, les polymères d'origine naturelle et leurs mélanges.
Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide.
Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résines toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.
Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences du polymère séquencé sont incompatibles l'une avec l'autre. De tels polymères sont décrits par exemple dans les documents EP 1411069 ou WO04/028488.
Le polymère filmogène additionnel peut être présent dans la composition selon l'invention en une teneur allant de 0,1 % à 60 % en poids, par rapport au poids total de la composition, de préférence allant de 2 % à 40 % en poids, et mieux de 5 % à 25 % en poids.

Agent auxiliaire de filmification

Pour améliorer les propriétés filmogènes de la composition de vernis à ongles un agent auxiliaire de filmification peut être prévu.
Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).
Ainsi, la composition peut comprendre, en outre, au moins un agent plastifiant et/ou un agent de coalescence. En particulier, on peut citer, seuls ou en mélange, les plastifiants et agents de coalescence usuels, tels que :

- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, de tartrates, des phosphates, des sébaçates ;
- les esters issus de la réaction d'un acide monocarboxylique de formule $R_{11}COOH$ avec un diol de formule $HOR_{12}OH$ avec $R_{11}$ et $R_{12}$, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O , S, en particulier le monoester résultant de la réaction de l'acide isobutyrique et d'octanediol tel que le triméthyl-2,2,4 pentane diol 1,3, tel que celui commercialisé sous la référence TEXANOL Ester Alcohol par la société Eastman Chemical,
- des dérivés oxyéthylénés, tels que les huiles oxyéthylénées, notamment les huiles végétales, telles que l'huile de ricin ;
- les dimethicone copolyols, notamment à groupements □[ □ propyl polyoxypropylène et
- leurs mélanges.

Agent épaississant

La composition peut comprendre, outre le polymère polyoxyalkyléné, un agent épaississant qui peut en particulier être choisi parmi : les silices hydrophobes, telles que celles décrites dans le document EP-A-898960, et par exemple commercialisées sous les références "AEROSIL R812®" par la société Degussa, "CAB-O-SIL TS-530®", "CAB-O-SIL TS-610®", "CAB-O-SIL TS-720®"par la société Cabot, "AEROSIL R972®", "AEROSIL R974®" par la société Degussa, les silices hydrophiles telle que celle commercialisée sous la référence "AEROSIL 200®" par la société Degussa; les argiles telles que la montmorillonite, les argiles modifiées telles que les bentones comme par exemple, l'hectorite modifiée par un groupement stearyl benzyl alkyl ammonium, la bentonite modifiée par un groupement stearyl benzyl alkyl ammonium, les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La proportion totale en agent(s) épaississant(s) dans les compositions selon l'invention peut aller de à 0,01 à 15 % en poids, par rapport au poids total de la composition, de préférence de 0,1 à 10% et mieux de 0,1 à 15% en poids.

Selon un mode de réalisation particulier, la teneur totale en agents épaississants est inférieure ou égale à 5 % en poids, par rapport au poids total de la composition, de préférence inférieure ou égale à 3% en poids.

Selon un mode de réalisation particulier, la composition comprend un agent épaississant choisi parmi les bentones, en une teneur inférieure ou égale à 2% en poids par rapport au poids total de la composition, de préférence inférieure ou égale à 1% en poids.

Matière colorante

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica

titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Autres Additifs

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

Les exemples qui suivent illustrent l'invention. Sauf indication contraire, les quantités indiquées sont des pourcentages massiques.

**Exemples 1 et 2: Vernis à ongles**

On a préparé un vernis à ongle selon l'invention comprenant un polymère polyoxyalkylèné (Elfacos T 212 d'Akzo Nobel) et 0,64% de bentone et un vernis à ongles selon l'art antérieur comprenant 1,28% de bentone et ne comprenant pas de polymère polyoxyalkylèneé.

| | Exemple 1 selon l'invention | Exemple 2 hors invention |
|---|---|---|
| Nitrocellulose à 30 % d'alcool isopropylique (viscosité: E22 - 1/2 S) | 11,97 | 12,58 |
| Nitrocellulose à 30 % d'alcool isopropylique (Azur E80 de Bergerac) | 2,86 | 2,58 |
| Résine alkyde glycérophtalique ésterifiée par acides gras ramifiés(1) dans l'acétate d'éthyle à 70 % | 15,29 | 13,8 |

(suite)

| | Exemple 1 selon l'invention | Exemple 2 hors invention |
|---|---|---|
| Acétyle citrate de tributyle | 1,69 | 1,52 |
| N-éthyl O,P-toluènesulfonamide | 4,80 | 4,34 |
| Hectorite modifiée stearyl benzyl dimethyl ammonium (BENTONE 27V d'Elementis) | 0,64 | 1,28 |
| PPG-14 palmeth-60 hexyl dicarbamate (ELFACOS T 212 d'Akzo Nobel) | 0,25 | - |
| Alcool isopropylique | 3,59 | 3,49 |
| Acide citrique, 1H2O | 0,026 | 0,05 |
| Acétate d'éthyle | 22,44 | 20,21 |
| Acétate de butyle | Qsp 100 | Qsp 100 |

(1) commercialisé par SHELL sous le nom de marque CARDURA 30®.

On a mesuré la brillance des deux vernis selon le protocole suivant :
Sur une carte de contraste de marque LENETA et de référence FORM 1A PENOPAC, on étale une couche de 300 µm d'épaisseur humide de la composition dont on cherche à évaluer la brillance moyenne, à l'aide d'un étaleur automatique. La couche recouvre le fond blanc et la fond noir de la carte. On laisse sécher pendant 24 heures sur un banc thermostaté à 30°C puis on procède à la mesure de la brillance à 20° et à 60° sur le fond blanc (3 mesures) et sur le fond noir (3 mesures) à l'aide d'un brillancemètre de marque BYK GARDNER et de référence microTRI-GLOSS.

Les résultats sont présentés dans le tableau suivant :

| Brillance (%) | Exemple 1 | Exemple 2 |
|---|---|---|
| 20° | 76 | 52 |
| 60° | 91 | 83 |

Le vernis à ongles de l'exemple 1 selon l'invention est stable, il s'applique facilement sur l'ongle et forme un film de brillance plus élevée que le vernis de l'exemple 2 selon l'art antérieur.

## Revendications

1. Composition de vernis à ongles comprenant, dans un milieu cosmétiquement acceptable comprenant un milieu solvant organique, au moins 0,1% d'au moins un polymère ayant un squelette soluble dans le milieu solvant organique, ledit polymère étant choisi parmi les polymères de structure $R_9$-(ABA)n-$R_9$ dans laquelle:

   les radicaux $R_9$ représentent indépendamment un atome d'hydrogène, un groupe alkyle ou aryle comprenant de 6 à 18 atomes de carbone,
   A représente une chaîne polyoxylakylène, de préférence comprenant des groupes polyoxyethylène (POE), une chaîne hydrocarbonée comprenant des groupes polyoxyalkylènés, une chaîne de copoly(oxyethylène/oxypropylène),
   B représente un groupement -CO-NH-$R_{10}$-NH-CO- dans lequel $R_{10}$ est une chaîne hydrocarbonée linéaire ou cyclique, de préférence un groupe alkyle comprenant de 4 à 12 atomes de carbone et
   n est un entier allant de 1 à 10,

   et au moins un agent épaississant.

2. Composition la revendication précédente, **caractérisée en ce que** le polymère est choisi parmi les polymères obtenus par réaction d'hexaméthylène diisocyanate et d'au moins un copolymère bloc polyoxyéthylène / polyoxypropylène.

3. Composition l'une des revendications précédentes, **caractérisée en ce que** le polymère est choisi parmi les polymères de formule suivante :

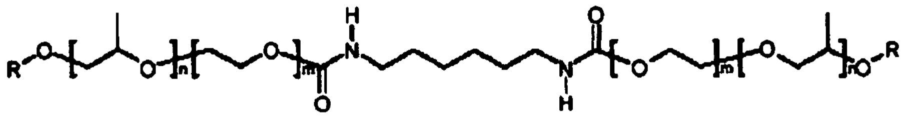

dans laquelle :

les radicaux R, identiques ou différents représentent un groupement alkyle comprenant de 6 à 20 atomes de carbone,
m représente un nombre entier allant de 1 à 100 et
n représente un nombre entier allant de 1 à 200.

**4.** Composition selon l'une des revendications précédentes, **caractérisée en ce que** le polymère est un polymère de diuréthane (hexamethylène diisocyanate) d'alcools en C16-C18 oxyethylénés 66 OE et oxypropylénés 14 OP.

**5.** Composition selon l'une des revendications précédentes, **caractérisée en ce que** le polymère polyoxyalkyléné est présent en une quantité allant de 0,1 à 15% en poids par rapport au poids total de la composition, de préférence de 0,5 à 10%, mieux de 1 à 8% en poids et encore mieux de 1 à 5% en poids.

**6.** Composition selon l'une des revendications précédentes, **caractérisée en ce que** le milieu solvant organique comprend au moins un solvant organique choisi parmi :

- les cétones liquides à température ambiante tels que les méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la $\gamma$-butyrolactone ;
- les esters à chaîne courte ayant de 3 à 8 atomes de carbone au total tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle,
- les acétales tels que le méthylal ;
- leurs mélanges.

**7.** Composition selon l'une des revendications 1 à 6, **caractérisée en ce que** l'agent épaississant est choisi parmi les silices hydrophobes, les silices hydrophiles, les argiles telles que la montmorillonite, les argiles modifiées telles que les bentones, les alkyléther de polysaccharides et leurs mélanges.

**8.** Composition selon l'une des revendications 1 à 7, **caractérisée en ce que** la teneur totale en agents épaississants est inférieure ou égale à 5 % en poids, par rapport au poids total de la composition, de préférence inférieure ou égale à 3% en poids.

**9.** Composition selon l'une des revendications précédentes, **caractérisée en ce qu'**elle comprend un agent épaississant choisi parmi les bentones, en une teneur inférieure ou égale à 2% en poids par rapport au poids total de la composition.

**10.** Composition selon l'une des revendications précédentes, **caractérisée en ce qu'**elle comprend un polymère filmogène.

**11.** Composition selon l'une des revendications précédentes, **caractérisée en ce qu'**elle comprend une matière colorante.

**12.** Procédé cosmétique de maquillage ou de soin non thérapeutique des ongles comprenant l'application sur les ongles d'au moins une couche d'une composition de vernis à ongles selon l'une des revendications 1 à 11.

**13.** Utilisation dans une composition de vernis à ongles selon l'une des revendications 1 à 11 d'un polyuréthane selon la revendication 1 pour obtenir déposé sur les ongles un vernis à ongles présentant une bonne stabilité et une bonne homogénéité de la couleur dans le temps, ainsi que la formation d'un film sur les ongles brillant et de bonne tenue.

**14.** Ensemble cosmétique comprenant : i) un récipient délimitant au moins un compartiment, ledit récipient étant fermé par un élément de fermeture et ii) une composition disposée à l'intérieur dudit compartiment, la composition étant conforme à l'une quelconque des revendications 1 à 11.

## Claims

**1.** Nail varnish composition comprising, in a cosmetically acceptable medium comprising an organic solvent medium, at least 0.1% of at least one polymer having a backbone that is soluble in the organic solvent medium, the said polymer being chosen from polymers of structure $R_9$-(ABA)n-$R_9$ in which:

the radicals $R_9$ independently represent a hydrogen atom or an alkyl or aryl group containing from 6 to 18 carbon atoms,
A represents a polyoxyalkylenated chain, preferably comprising polyoxyethylene (POE) groups, a hydrocarbon-based chain comprising polyoxyalkylenated groups or a copoly(oxyethylene/oxypropylene) chain,
B represents a group -CONH$R_{10}$NHCO- in which $R_{10}$ is a linear or cyclic hydrocarbon-based chain, preferably an alkyl group containing from 4 to 12 carbon atoms, and
n is an integer ranging from 1 to 10,

and at least one thickener.

**2.** Composition according to the preceding claim, **characterized in that** the polymer is chosen from polymers obtained by reaction of hexamethylene diisocyanate and of at least one polyoxyethylene/polyoxypropylene block copolymer.

**3.** Composition according to either of the preceding claims, **characterized in that** the polymer is chosen from polymers having the following formula:

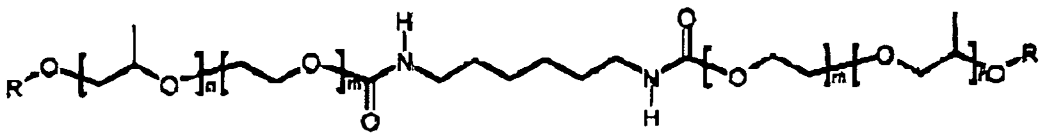

in which:

the radicals R, which may be identical or different, represent an alkyl group containing from 6 to 20 carbon atoms,
m represents an integer ranging from 1 to 100, and
n represents an integer ranging from 1 to 200.

**4.** Composition according to one of the preceding claims, **characterized in that** the polymer is a 66 EO oxyethylenated and 14 PO oxypropylenated C16 C18 alcohol diurethane (hexamethylene diisocyanate) polymer.

**5.** Composition according to one of the preceding claims, **characterized in that** the polyoxyalkylenated polymer is present in an amount ranging from 0.1% to 15% by weight, preferably from 0.5% to 10%, better still from 1% to 8% and even better still from 1% to 5% by weight relative to the total weight of the composition.

**6.** Composition according to one of the preceding claims, **characterized in that** the organic solvent medium comprises

at least one organic solvent chosen from:

- ketones that are liquid at room temperature, such as methyl ethyl ketone, methyl isobutyl ketone, diisobutyl ketone, isophorone, cyclohexanone or acetone;
- alcohols that are liquid at room temperature, such as ethanol, isopropanol, diacetone alcohol, 2butoxyethanol or cyclohexanol;
- propylene glycol ethers that are liquid at room temperature, such as propylene glycol monomethyl ether, propylene glycol monomethyl ether acetate or dipropylene glycol mono-n-butyl ether;
- cyclic ethers such as γ-butyrolactone;
- short-chain esters containing from 3 to 8 carbon atoms in total such as ethyl acetate, methyl acetate, propyl acetate, isopropyl acetate, nbutyl acetate, isopentyl acetate, methoxypropyl acetate or butyl lactate;
- ethers that are liquid at room temperature, such as diethyl ether, dimethyl ether or dichlorodiethyl ether;
- alkanes that are liquid at room temperature, such as decane, heptane, dodecane or cyclohexane;
- alkyl sulfoxides such as dimethyl sulfoxide;
- aldehydes that are liquid at room temperature, such as benzaldehyde or acetaldehyde;
- ethyl 3-ethoxypropionate;
- carbonates such as propylene carbonate or dimethyl carbonate;
- acetals such as methylal;
- and mixtures thereof.

**7.** Composition according to one of Claims 1 to 6, **characterized in that** the thickener is chosen from hydrophobic silicas, hydrophilic silicas, clays such as montmorillonite, modified clays such as bentones, and polysaccharide alkyl ethers, and mixtures thereof.

**8.** Composition according to one of Claims 1 to 7, **characterized in that** the total thickener content is less than or equal to 5% by weight, preferably less than or equal to 3% by weight relative to the total weight of the composition.

**9.** Composition according to one of the preceding claims, **characterized in that** it comprises a thickener chosen from bentones, in a content of less than or equal to 2% by weight relative to the total weight of the composition.

**10.** Composition according to one of the preceding claims, **characterized in that** it comprises a filmforming polymer.

**11.** Composition according to one of the preceding claims, **characterized in that** it comprises a dyestuff.

**12.** Non-therapeutic cosmetic process for making up or caring for the nails, comprising the application to the nails of at least one coat of a nail varnish composition according to one of Claims 1 to 11.

**13.** Use, in a nail varnish composition, according to one of Claims 1 to 11, of a polyurethane according to Claim 1, to obtain a deposit on the nails of a nail varnish that shows good stability and good colour homogeneity over time, and also the formation of a glossy film with good staying power on the nails.

**14.** Cosmetic assembly comprising: i) a container delimiting at least one compartment, the said container being closed by means of a closing member, and ii) a composition placed inside the said compartment, the composition being in accordance with any one of Claims 1 to 11.

## Patentansprüche

**1.** Nagellackzusammensetzung, die in einem kosmetisch verträglichen Medium, das ein organisches Lösemittelmedium umfasst, mindestens 0,1 % mindestens eines Polymers umfasst, das ein Gerüst aufweist, welches in dem organischen Lösemittelmedium löslich ist, wobei das Polymer ausgewählt ist aus den Polymeren der Struktur $R_9$-(ABA)n-$R_9$, wobei:

die Radikale $R_9$ unabhängig voneinander ein Wasserstoffatom, eine Alkyl- oder eine Arylgruppe darstellen, die 6 bis 18 Kohlenstoffatome umfasst,
A eine Polyoxyalkylenkette darstellt, die vorzugsweise Polyoxyethylengruppen (POE), eine Polyoxyalkylengruppen umfassende Kohlenwasserstoffkette, eine Copolyoxyethylen/oxypropylenkette umfasst,
B eine Gruppierung -CO-NH-$R_{10}$-NH-CO-darstellt, wobei $R_{10}$ eine lineare oder cyclische Kohlenwasserstoffkette

ist, vorzugsweise eine Alkylgruppe, die 4 bis 12 Kohlenstoffatome umfasst sowie
n eine ganze Zahl zwischen 1 und 10 ist

sowie mindestens ein Verdickungsmittel.

**2.** Zusammensetzung nach dem vorherigen Anspruch, **dadurch gekennzeichnet, dass** das Polymer ausgewählt ist aus den Polymeren, die aus der Reaktion von Hexamethylendiisocyanat mit mindestens einem Polyoxyethylen/Polyoxypropylen-Blockcopolymer hervorgeht.

**3.** Zusammensetzung nach einem der vorherigen Ansprüche, **dadurch gekennzeichnet, dass** das Polymer ausgewählt ist aus den Polymeren der folgenden Formel:

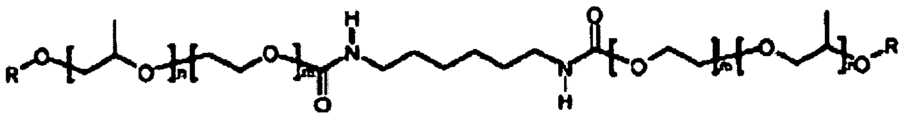

wobei:

die Radikale R, identisch oder voneinander verschieden, eine Alkylgruppe darstellen, die 6 bis 20 Kohlenstoffatome umfasst,
m eine ganze Zahl von 1 bis 100 darstellt und
n eine ganze Zahl zwischen 1 bis 200 darstellt.

**4.** Zusammensetzung nach einem der vorherigen Ansprüche, **dadurch gekennzeichnet, dass** das Polymer ein Diurethanpolymer (Hexamethylendiisocyanat) von C16-C18 Oxyethylenalkoholen 66 OE und Oxypropylenalkoholen 14 OP ist.

**5.** Zusammensetzung nach einem der vorherigen Ansprüche, **dadurch gekennzeichnet, dass** das Polyoxyalkylenpolymer in einer Menge von 0,1 bis 15 Gew.% bezogen auf das Gesamtgewicht der Zusammensetzung vorliegt, vorzugsweise von 0,5 bis 10 Gew.%, weiter bevorzugt von 1 bis 8 Gew.% und insbesondere von 1 bis 5 Gew.%.

**6.** Zusammensetzung nach einem der vorherigen Ansprüche, **dadurch gekennzeichnet, dass** das organische Lösemittelmedium mindestens ein organisches Lösemittel umfasst, das ausgewählt ist aus:

- bei Raumtemperatur flüssigen Ketonen, wie etwa Methylethylketon, Methylisobutylketon, Diisobutylketon, Isophoron, Cyclohexanon, Aceton;
- bei Raumtemperatur flüssigen Alkoholen, wie etwa Ethanol, Isopropanol, Diacetonalkohol, 2-Butoxyethanol, Cyclohexanol;
- bei Raumtemperatur flüssigen Propylenglykolethern; wie etwa Propylenglykolmonomethylether; Propylenglykolmonomethyletheracetat; Dipropylenglykolmono-n-butylether;
- cyclischen Ethern, wie etwa γ-Butyrolacton;
- kurzkettigen Estern, die insgesamt 3 bis 8 Kohlenstoffatome aufweisen, wie etwa Ethylacetat, Methylacetat, Propylacetat, Isopropylacetat, n-Butylacetat, Isopentylacetat, Methoxypropylacetat, Butyllactat;
- bei Raumtemperatur flüssigen Ethern, wie etwa Diethylether, Dimethylether oder Dichlorodiethylether;
- bei Raumtemperatur flüssigen Alkanen, wie etwa Decan, Heptan, Dodecan, Cyclohexan;
- Alkylsulfoxiden, wie etwa Dimethylsulfoxid;
- bei Raumtemperatur flüssigen Aldehyden, wie etwa Benzaldehyd, Acetaldehyd;
- Ethyl-3-ethoxypropionat;
- Carbonaten, wie etwa Propylencarbonat, Dimethylcarbonat;
- Acetalen, wie etwa Methylal;
- deren Gemischen.

**7.** Zusammensetzung nach einem der Ansprüche 1 bis 6, **dadurch gekennzeichnet, dass** das Verdickungsmittel ausgewählt ist aus hydrophoben Kieselsäuren, hydrophilen Kieselsäuren, Tonarten, wie etwa Montmorillonit, modifizierten Tonarten, wie etwa Bentonit, Polysaccharidalkylethern und deren Gemischen.

8. Zusammensetzung nach einem der Ansprüche 1 bis 7, **dadurch gekennzeichnet, dass** der Gesamtgehalt an Verdickungsmitteln kleiner oder gleich 5 Gew.% bezogen auf das Gesamtgewicht der Zusammensetzung ist, vorzugsweise kleiner oder gleich 3 Gew.%.

9. Zusammensetzung nach einem der vorherigen Ansprüche, **dadurch gekennzeichnet, dass** sie ein Verdickungsmittel umfasst, das ausgewählt ist aus Bentoniten zu einem Gehalt von kleiner oder gleich 2 Gew.% bezogen auf das Gesamtgewicht der Zusammensetzung.

10. Zusammensetzung nach einem der vorherigen Ansprüche, **dadurch gekennzeichnet, dass** sie ein filmbildendes Polymer umfasst.

11. Zusammensetzung nach einem der vorherigen Ansprüche, **dadurch gekennzeichnet, dass** sie ein Färbemittel umfasst.

12. Kosmetisches Verfahren zum Make-up oder zur nicht therapeutischen Pflege von Fingernägeln, das die Anwendung mindestens einer Schicht einer Nagellackzusammensetzung nach einem der Ansprüche 1 bis 11 auf die Nägel umfasst.

13. Verwendung eines Polyurethans nach Anspruch 1 in einer Nagellackzusammensetzung nach einem der Ansprüche 1 bis 11, zum Erhalt einer Nagellackschicht auf den Nägeln, die eine gute zeitliche Stabilität und eine gute Farbhomogenität aufweist ebenso wie die Ausbildung eines Films auf den Nägeln, der schimmernd und von gutem Halt ist.

14. Kosmetische Einheit, umfassend: i) einen Behälter, der mindestens eine Kammer umgrenzt, wobei der Behälter von einem Verschlusselement verschlossen ist sowie ii) eine im Inneren der Kammer angeordnete Zusammensetzung, wobei die Zusammensetzung einer derjenigen der Ansprüche 1 bis 11 entspricht.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6106820 A
- US 4104333 A
- US 4229227 A
- EP 1495748 A
- EP 1411069 A
- WO 04028488 A
- EP 898960 A
- EP 898958 A